# EUROPEAN PATENT APPLICATION

(11) **EP 1 479 772 A1**
(43) Date of publication of application: **24.11.2004**
(21) Application number: 03705317.0
(22) Date of filing: 19.02.2003
(51) Int. Cl.: C12N 15/11, C07K 14/47, C12Q 1/02, C12Q 1/68, A61K 45/00, A61P 25/02, A61P 25/14, A61P 25/16, A61P 25/28, A61P 35/00, A61P 37/02, A61P 43/00, G01N 33/15, G01N 33/50

(54) **APOPTOSIS PROMOTER AND INHIBITOR INTERACTING WITH CGI-94 AND METHOD OF SCREENING THE SAME**

(30) Priority: 19.02.2002 JP 2002041176
(71) Applicant: BF Research Institute, Inc., Osaka-shi, Osaka 532-8686 (JP)
(72) Inventor: HEESE, Klaus, Minoh-shi, Osaka 562-0025 (JP); YAMADA, Takashi, Minoh-shi, Osaka 562-0032 (JP); NAGAI, Yasuo, Minoh-shi, Osaka 562-0005 (JP); SAWADA, Tohru, Ibaraki-shi, Osaka 567-0064 (JP)
(74) Representative: Walton, Seán Malcolm
(86) International application number: PCT/JP2003/001778
(87) International publication number: WO 2003/070947

(57) **Abstract**

The present invention provides substances which suppress or promote apoptosis of nerve cells, methods for screening them, and methods for diagnosis and treatment of diseases associated with apoptosis and kits therefore. Specifically, the present invention provides suppress or promote functions of CGI-94 protein or the interaction of CGI-94 protein with Smac/DIABLO to suppress or promote apoptosis. In addition, the present invention also provides a p18TFP protein and its coding DNA as an apoptosis suppressing substance, and pharmaceutical compositions for treating diseases associated with apoptosis, which comprises said protein or DNA.

## Description

### TECHNICAL FIELD OF THE INVENTION

The present invention relates to apoptosis suppressing or promoting substances, their screening, and diagnosis and treatment of diseases associated with apoptosis. More particularly, the present invention relates to substances which interact with Smac/DIABLO involved in apoptosis and suppress or promote apoptosis through direct interaction with CGI-94 causing apoptosis or through interaction with Smac/DIABLO, in particular, an apoptosis suppressing protein, p18TFP, and its coding gene. The present invention further relates to applications to diagnosis of cells or tissues affected due to changes in their expression.

### BACKGROUND

Apoptosis of cells is not only physiological changes seen in the growth stage, such as, in inducing cell differentiation (Bri. J. Cancer, 26, 239 (1972)), but also a phenomenon in which its abnormalities are observed in diseased processes of neurodegenerative disorders such as Alzheimer's disease (Acta. Neuropathol., 89, 35 (1995); J. Neurol. Sci., 152, 73 (1997)), cancers, and others (Bri. J. Cancer, 26, 239 (1972)). In particular, increased unusual apoptosis (Science, 243, 535 (1989); Cell, 97, 395 (1999); Nature, 399, 263 (1999)) or suppressed apoptosis (Adv. Immunol., 50, 55 (1991)) results in loss of persistence of integral functions in the body and will lead to serious diseases. Recently, studies on proteins involved in apoptosis and on their physiological and pathological mechanisms (Genes Dev., 10, 1 (1996); Cell, 87, 171 (1996); Nature, 387, 773 (1997); Science, 275, 1122 (1997); Cancer Res., 62, 18 (2002)) are progressing dramatically by the progression of molecular biological researches. However, there is still a lot of uncertainty on its detailed mechanism. Elucidating the mechanism of apoptosis and controlling apoptosis are clearly very useful in medical treatment of various diseases, including Alzheimer's disease and cancers, and are therapeutical targets (Science, 267, 1456 (1995); Ann. Neurol., 38, 839 (1995); Trends Pharmacol. Sci., 20, 35 (1999)).

### Problems to be Solved by the Invention

The inventors of the present invention have found that the expression of a gene CGI-94 is suppressed in the hippocampus of brains obtained from patients having early stage of Alzheimer's disease (Eur. J. Neurosci. 15, 79 (2002)). At present, however, physiological and pathological roles of the protein produced by this gene are not known at all (Genome Res., 10, 703 (2000)). Thus, in order to elucidate functions of the protein (CGI-94), the inventors of the present invention have conducted extensive research, in which a protein having a fluorescent protein GFP (Green fluorescent protein) fused at the C-terminal (CGI-94-CT-GFP) was expressed in PC12 cells, whose differentiation was induced with NGF (Nerve growth factor). As a result, 120 hours after adding NGE, neurite elongation was suppressed and apoptosis was observed in cells expressing CGI-94-GFP. In addition, two weeks later, all cells were found to be dead. On the other hand, in unxepressing cells, neurite elongation and survival by the addition of NGF were observed. Thus, it is likely that CGI-94 gives rise to neurite-elongation suppression and apoptosis. Although the suppression of expression of CGI-94 at early stages in a brain having early stage of Alzheimer's disease and the cell-death promoting effect seem to be apparently a contradictory event, such an event is probably due to an apoptosis suppressing function in the brain having early stage of Alzheimer's disease.

In addition, the inventors of the present invention utilized a method described as the Two-Hybrid System (Proc. Natl. Acad. Sci. USA, 88, 9578 (1991)) and searched proteins interacting with this CGI-94, and consequently have shown that a protein involved in cell apoptosis, Smac/DIABLO, displays a strong interaction with CGI-94. It has been reported that Smac/DIABLO is released into the cell with cytochrome c from mitochondria during apoptosis, and promotes apoptosis downstream of cytochrome c by inhibiting effects of inhibitory apoptosis proteins (IAPs), such as XIAP, c-IAP, c-IAP2, or survivin, and further promoting caspase-3 activity (Cell, 102, 33 (2000); Cell, 102, 43 (2000); Nature, 406, 406, 855 (2000)). Thus, it is believed that in PC12 cells whose differentiation has been induced by NGF, CGI-94 gives rise to neurite-elongation suppression and apoptosis through the interaction with this Smac/DIABLO.

From the above-mentioned facts, substances interacting with CGI-94 to suppress its function result in suppressing of neurite-elongation suppression and of apoptosis, and are believed to suppress nerve-cell death in diseases associated with apoptosis of cells, particularly, of nerve cells, for example, Alzheimer's disease, and to control its progression. Also, substances promoting CGI-94 function promote apoptosis, and are believed to result in rapid enhancement, in particular, of cell apoptosis, and to be effective for treating, especially, diseases resulting from cell hyperproliferation, such as cancers and autoimmune disorders.

Furthermore, substances suppressing the interaction of CGI-94 with Smac/DIABLO result in suppressing of neurite-elongation suppression and of apoptosis, and are believed to suppress nerve-cell death in diseases associated with apoptosis of nerve cells, for example, Alzheimer' s disease, and to control its progression. Also, substances accelerating the interaction of CGI-94 with Smac/DIABLO promote apoptosis, and are believed to result in rapid enhancement of cell apoptosis, and to be effective for treating, especially, diseases resulting from cell hyperproliferation, such as cancers and autoimmune disorders.

### Means to Solve the Problems

Therefore, the present invention provides:
(1) a substance which interacts with CGI-94 to suppress apoptosis;
(2) a substance which suppresses the interaction of CGI-94 with Smac/DIABLO to suppress apoptosis;
(3) a DNA characterized by:
   (a) a DNA comprising the base sequence of base numbers 140 to 601 of the base sequence of SEQ ID NO:1,
   (b) a DNA hybridizable under stringent conditions with a DNA comprising the sequence of (a), or
   (c) a DNA having a homology of not less than 60% to the DNA sequence of (a);
(4) a protein characterized by:
   (a) a protein comprising of amino acids 1 to 153 of the amino acid sequence of SEQ ID NO:2 and suppressing apoptosis by interacting with CGI-94 or by suppressing the interaction of CGI-94 with Smac/DIABLO,
   (b) a protein having one or more amino acids deleted, substituted, or added in the amino acid sequence of (a) and suppressing apoptosis by interacting with CGI-94 or by suppressing the interaction of CGI-94 with Smac/DIABLO, or
   (c) a protein having a homology of not less than 60% to the amino acid sequence of (a);
(5) a DNA coding for the protein according to (4);
(6) a RNA which is complementary to the DNA according to (3) or (5);
(7) a substance which interacts with CGI-94 to promote apoptosis;
(8) a substance which promotes the interaction of CGI-94 with Smac/DIABLO to promote apoptosis;
(9) a method for screening substances interacting with CGI-94 to suppress its function, which comprises determining neurite elongation or apoptosis of a cell expressing CGI-94 in the presence of a test substance and a differentiation inducing factor;
(10) a method for screening substances interacting with CGI-94 to promote its function, which comprises determining neurite elongation or apoptosis of a cell expressing CGI-94 in the presence of a test substance and a differentiation inducing factor;
(11) a substance interacting with CGI-94 to suppress its function, wherein the substance is found by means of the method according to (9);
(12) a substance interacting with CGI-94 to promote its function, wherein the substance is found by means of the method according to (10);
(13) a method for screening substances suppressing the interaction of CGI-94 with Smac/DIABLO, which comprises determining neurite elongation or apoptosis of a cell expressing CGI-94 and Smac/DIABLO in the presence of a test substance and a differentiation inducing factor;
(14) a method for screening substances promoting the interaction of CGI-94 with Smac/DIABLO, which comprises determining neurite elongation or apoptosis of a cell expressing CGI-94 and Smac/DIABLO in the presence of a test substance and a differentiation inducing factor;
(15) a substance suppressing the interaction of CGI-94 with Smac/DIABLO, wherein the substance is found by means of the method according to (13);
(16) a substance according to (12) or (15), which is a p18TFP protein;
(17) a substance promoting the interaction of CGI-94 with Smac/DIABLO, wherein the substance is found by means of the method according to (14);
(18) a pharmaceutical composition for treating a disease associated with apoptosis, comprising a substance interacting with CGI-94 to suppress apoptosis;
(19) a pharmaceutical composition for treating a disease associated with apoptosis, comprising a substance suppressing the interaction of CGI-94 with Smac/DIABLO to suppress apoptosis;
(20) a pharmaceutical composition according to (18) or (19), wherein the substance is a p18TFP protein or a p18TFP DNA;
(21) a pharmaceutical composition for treating a disease resulting from cell hyperproliferation, comprising a substance interacting with CGI-94 to promote apoptosis;
(22) a pharmaceutical composition for treating a disease resulting from cell hyperproliferation, comprising a substance promoting the interaction of CGI-94 with Smac/DIABLO to promote apoptosis;
(23) a method for diagnosis of a disease associated with apoptosis, which comprises determining the level of expression of the CGI-94 gene or the CGI-94 protein in cells or tissues; and
(24) a kit for diagnosis of a disease associated with apoptosis, which comprises determining the level of expression of the CGI-94 gene or the CGI-94 protein in cells or tissues.

### BRIEF DESCRIPTION OF THE DRAWINGS

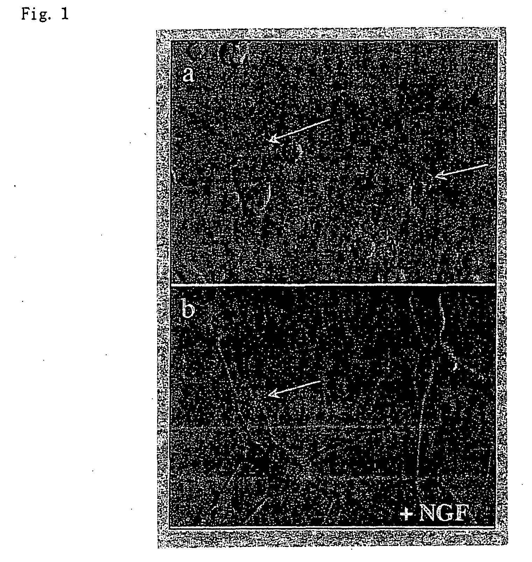

Fig. 1 shows fluorescent microphotographs of PC12 cells expressing the CGI-94-GFP fusion protein. Fig. 1a shows a photograph taken 48 hours after CGI-94-GFP was transiently expressed, indicating that all PC12 cells survived and that since NGF was not added, no elongation of neurites was observed under a fluorescence microscope. Fig. 1b indicates that when NGF (50 ng/ml) was added 24 hours after the expression and an additional 120 hours passed, cells not expressing the protein resulted in elongation of neurites, whereas cells expressing the protein (indicated by an arrow) resulted in suppressed elongation of neurites and apoptosis was observed under a fluorescence microscope.

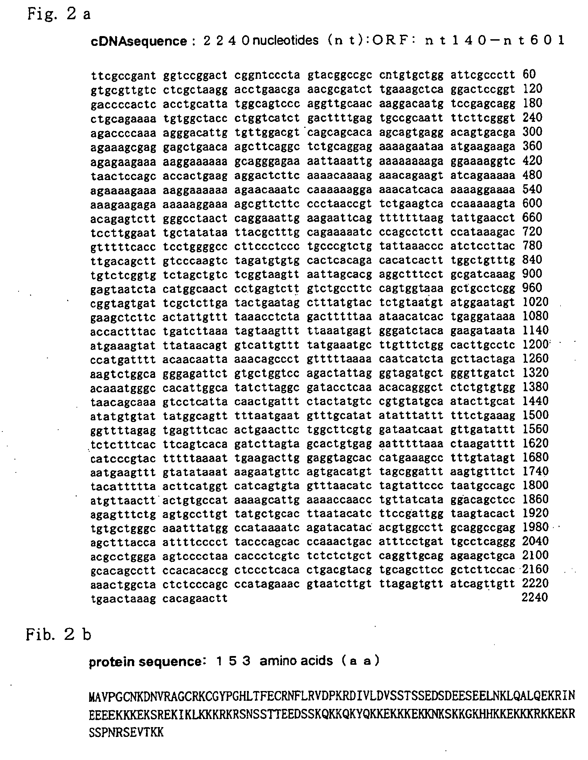

Fig. 2a shows the cDNA sequence of the ORF of an apoptosis suppressing protein p18TFP (also see, SEQ ID NO:1). Figure 2b shows the amino acid sequence of an apoptosis suppressing protein p18TFP (also see, SEQ ID NO:2).

### EMBODIMENTS FOR CARRING OUT THE INVENTION

In one aspect, the present invention relates to a substance which interacts with CGI-94 to suppress its function. As described above, a substance which interacts with CGI-94 to suppress its function results in suppressing of cell apoptosis, in particular, suppressing of neurite-elongation suppression and of apoptosis in nerve cells, and is useful for prophylaxis and/or treatment of encephalic neurodegenerative diseases associated with apoptosis of cells, particularly, of nerve cells, such as Alzheimer's disease, Down's syndrome, and other dementias, as well as Huntington's chorea disease, amyotrophic lateral sclerosis, spinocerebellar degenerative disease, Parkinson's disease, and the like. A substance which interacts with CGI-94 to suppress its function may be a substance obtainable by means of screening as described below. Substances which interact with CGI-94 to suppress its function can be any kind of molecules, including proteins, peptides, small molecules such as other low molecular-weight organic compounds.

In another aspect, the present invention further relates to a method for screening substances which interact with CGI-94 to suppress its function. Examples of methods for screening substances which interact with CGI-94 to suppress its function include methods comprising inserting a gene coding for a CGI-94 protein into an appropriate vector, transforming a cell using it, preferably a nerve cell, and then culturing the transformed cell under the action of a differentiation inducing factor (for example, NGF) in the presence of a test substance, and in the case of a nerve cell, assaying neurite elongation and apoptosis, and comparing to the result obtained without the test substance and/or without the differentiation inducing factor (i.e., a control system). A cell expressing CGI-94, also in the presence of a differentiation inducing factor at a given concentration, suppresses the elongation of neurites (in the case of a nerve cell), and apoptosis can be observed. On the other hand, the presence of a substance which interacts with CGI-94 to suppress its function allows growth of neurites of cells (in the case of a nerve cell), resulting in avoiding apoptosis. In such a case, it is likely that the test substance is an apoptosis suppressing substance which interacts with CGI-94 to suppress its function and suppress apoptosis. In order to evaluate whether apoptosis has been caused, it is usual to employ microscopic observations. When apoptosis takes place, there are observed, for example, cell condensation, vacuolation, surface smoothing, fragmentation of the cell and the nucleus and the like. Vectors which may be used for inserting the CGI-94 gene to transform a nerve cell may utilize a variety of vectors described in the literature and various commercial available vectors, for example, pIND, pIND/V5-His, pIND/GFP, pcDNA3.1 or pcDNA3.1/myc or pcDNA3.1/His or pcDNA3.1/V5-His or pcDNA3.1-CT-GFP-TOPO®, or virus vectors such as LNCX2. Methods for transformation are known, such as, calcium phosphate methods, Super Fector reagent methods, and lipid-mediated methods employing LipofectAMINE reagent or the like. Cells to be used for screening may utilize any cell, preferably nerve cells. Examples of preferred nerve cells include PC12 cell, NB2a, Neuro-2A, B104, SHSY-5Y, primary culture nerve cells, and the like. Conditions for culturing cells are determined depending upon respective cells, and are within the knowledge of those skilled in the art or can be readily determined. The nucleotide sequence of the CGI-94 gene and the amino acid sequence of the CGI-94 protein are known (Eur. J. Neurosci. 15, 79 (2001)). It is desirable to attach a detectable tag to a CGI-94 protein to be expressed, so that the expression of the CGI-94 protein can be detected with ease. Detectable tags include green fluorescent protein, red fluorescent protein, myc, and the like: fusions with green fluorescent protein, in particular, are simple and sensitive, and thus may be recommended.

The inventors of the present invention have found a p18TFP protein and its coding DNA as a substance which interacts with CGI-94 to suppress its function, by employing the above-described screening.

In one aspect of the present invention, therefore, a new DNA is provided which comprises the base sequence of base numbers 140 to 601 of the base sequence of SEQ ID NO:1. This DNA codes for p18TFP, a new protein of the present invention as shown in SEQ ID NO:2 (amino acids 1 to 153). Therefore, the p18TFP protein typically comprises the amino acid sequence of SEQ ID NO:2.

The present invention also includes a DNA hybridizable under stringent conditions with a DNA comprising the base sequence of base numbers 140 to 601 of the base sequence of SEQ ID NO:1, since it is likely that such a DNA has a similar structure, function, and the like to a DNA comprising the base sequence of base numbers 140 to 601 of the base sequence of SEQ ID NO:1. Stringent conditions are exemplified by conditions allowing hybridization at 42°C in a solution containing 6X SSC, 5X Denhardt's reagent, 0.5% SDS, and 100 µg/ml salmon sperm DNA. In addition, the present invention also relates to a DNA having a homology of at least 60% or more, preferably 70% or more, more preferably 80% or more, most preferably 90% or more, to a DNA sequence comprising the base sequence of base numbers 140 to 601 of the base sequence of SEQ ID NO:1. Such a DNA also includes a human p18TFP DNA. The above-described DNAs/genes collectively may be referred to as p18TFP DNA/gene.

In another aspect, the present invention provides a new rat p18TFP protein comprising of the amino acid sequence (amino acids 1 to 153) of SEQ ID NO:2, and variant proteins thereof. As described above, this protein is typically a protein comprising of the amino acid sequence (amino acids 1 to 153) of SEQ ID NO:2, while the protein may be a variant protein comprising of the amino acid sequence having one or more amino acids deleted, substituted, or added in the amino acid sequence of SEQ ID NO:2. Also included are variant proteins having a homology of at least 60% or more, preferably 70% or more, more preferably 80% or more, most preferably 90% or more, to the amino acid sequence (amino acids 1 to 153) of SEQ ID NO:2, and such proteins also include p18TFP of a human. In this case, such variant proteins also have substantially the same activity as that of the new p18TFP comprising of the amino acid sequence (amino acids 1 to 153) of SEQ ID NO:2, that is, they have an apoptosis suppressing activity. In.the specification, the present p18TFP protein and variant proteins having substantially the same activity collectively may be referred to as p18TFP, or alternatively as p18TFP protein.

In one aspect, the present invention further relates to a DNA coding for the above-described p18TFP or a variant protein thereof. In another aspect, the present invention relates to a RNA complementary to the above-described p18TFP DNA, and also to a RNA complementary to a DNA coding for the above-described p18TFP and a variant protein thereof.

Furthermore, the present invention relates to a method for the treatment of a disease associated with apoptosis of cells, preferably nerve cells, characterized by administering to a subject a substance which interacts with CGI-94 to suppress its function. Furthermore, the present invention also relates to a pharmaceutical composition for the treatment of a disease associated with apoptosis of cells, preferably nerve cells, comprising a substance which interacts with CGI-94 to suppress its function, for example, a p18TFP protein, and pharmaceutically acceptable carriers or excipients. Methods for manufacturing pharmaceutical compositions, dosage forms, and pharmaceutically acceptable carriers or excipients are selected depending upon conditions of subjects, sites to be treated, routes of administration, and others, and can be readily selected by those skilled in the art. Also, the present invention provides a method for the treatment of apoptosis by gene therapy by which a DNA coding for a p18TFP protein is administered to cells of a subject to express the p18TFP protein, thereby suppressing CGI-94 function.

In another aspect, the present invention further relates to a substance which interacts with CGI-94 to promote its function. As described above, a substance which interacts with CGI-94 to promote its function allows intentional cell apoptosis to take place, and thus is useful for establishing apoptosis systems, research on apoptosis mechanism, various studies relating to apoptosis, and research on diseases associated with apoptosis of cells, particularly, of nerve cells, such as research on Alzheimer's disease, for example, for elucidating its developing mechanism, and others. A substances promoting CGI-94 function promotes apoptosis and it is likely that the substance results in rapid enhancement of cell apoptosis and is useful for treating diseases, in particular, diseases resulting from cell hyperproliferation such as cancers and autoimmune disorders in particular by promoting cell apoptosis rapidly. A substance which interacts with CGI-94 to promote its function may be a substance obtainable by means of screening as described below. Substances which interact with CGI-94 to promote its function can be any kind of molecules, including, for example, proteins, peptides, small molecules such as other low molecular-weight organic compounds.

In another aspect, the present invention further relates to a method for screening substances which interact with CGI-94 to promote its function. Examples of methods for screening substances which interact with CGI-94 protein to promote its function include methods comprising inserting a gene coding for CGI-94 into an appropriate vector, transforming a nerve cell using it, and then culturing the transformed cell under the action of a differentiation inducing factor in the presence of a test substance, assaying neurite elongation and apoptosis, and comparing to the result obtained in the absence of the test substance. When in the presence of a differentiation inducing factor at higher concentrations than employed in the screening of substances which interact with CGI-94 to suppress its functions, a test substance is added to a nerve cell having neurite elongation or a cell causing no apoptosis, whereby the growth of neurites is suppressed (in the case of a nerve cell) or apoptosis is observed, it is likely that the test substance is a substance which interacts with CGI-94 to enhance its function and to promote apoptosis. Vectors which may be used for inserting the CGI-94 gene to transform a nerve cell may utilize a variety of vectors described in the literature and commercial available vectors, for example, pIND, pIND/V5-His, pIND/GFP, pcDNA3.1 or pcDNA3.1/myc or pcDNA3.1/His or pcDNA3.1/V5-His or pcDNA3.1-CT-GFP-TOPO®, or virus vectors such as LNCX2. Methods for transformation are known, such as, calcium phosphate methods, Super Fector reagent methods, and lipid-mediated methods employing LipofectAMINE reagent or the like. Cells to be used for screening may utilize any cell, preferably nerve cells. Examples of preferred nerve cells include PC12 cell, NB2a, Neuro-2A, B104, SHSY-5Y, primary culture nerve cells, and the like. Conditions for culturing cells are determined depending upon respective cells, and are within the knowledge of those skilled in the art or can be readily determined. The nucleotide sequence of the CGI-94 gene and the amino acid sequence of the CGI-94 protein are known (Eur. J. Neurosci. 15, 79 (2001)). It is desirable to attach a detectable tag to a CGI-94 protein to be expressed, so that the expression of the CGI-94 protein can be detected with ease. Detectable tags include green fluorescent protein, red fluorescent protein, myc, and the like: fusions with green fluorescent protein, in particular, are simple and sensitive, and thus may be recommended.

In a further aspect, the present invention relates to a substance which suppresses the interaction of CGI-94 with Smac/DIABLO. The present invention further relates to a method for screening substances which suppress the interaction of CGI-94 with Smac/DIABLO. Examples of methods for screening substances which suppress the function of CGI-94 with Smac/DIABLO include methods comprising inserting a gene coding for a CGI-94 protein and a gene coding for Smac/DIABLO into an appropriate vector, transforming a nerve cell using them; and then culturing transformed cell under the action of a differentiation inducing factor in the presence of a test substance, assaying neurite elongation and apoptosis, and comparing to the result obtained in the absence of the test substance. A cell expressing the CGI-94 and Smac/DIABLO proteins, even in the presence of a differentiation inducing factor at a given concentration, suppresses the elongation of neurites (in the case of a nerve cell), and apoptosis can be observed. On the other hand, the presence of a substance which suppresses the interaction between CGI-94 and Smac/DIABLO proteins allows growth of neuritis of cells (in the case of a nerve cell), avoiding apoptosis. In such a case, it is likely that the test substance is a substance which suppresses the interaction between CGI-94 and Smac/DIABLO proteins to suppress apoptosis. Vectors may be used for inserting the CGI-94 and Smac/DIABLO genes to a nerve cell, methods for transformation, and cells to be used can utilize those as described above. Conditions for culturing cells are determined depending upon respective cells, and are within the knowledge of those skilled in the art or can be readily determined. The nucleotide sequence of the CGI-94 gene and the amino acid sequence of the CGI-94 protein are known (Eur. J. Neurosci. 15, 79 (2001)), and the nucleotide sequence of the Smac/DIABLO gene and the amino acid sequence of the Smac/DIABLO protein are also known (Cell, 102, 33 (2000) ; Cell, 102, 43 (2000)). It is desirable to attach a detectable tag to a CGI-94 or Smac/DIABLO protein to be expressed, so that the expression of the CGI-94 or Smac/DIABLO protein can be detected with ease. Detectable tags include green fluorescent protein, red fluorescent protein, myc, or the like: fusions with green fluorescent protein, in particular, are simple and sensitive and thus may be recommended.

As mentioned above, a substance which suppresses the interaction of CGI-94 with Smac/DIABLO results in suppressing of neurite-elongation suppression and of apoptosis, and is useful for prophylaxis and/or treatment of encephalic neurodegenerative diseases associated with apoptosis of nerve cells, such as Alzheimer's disease, Down's syndrome, and other dementias, as well as Huntington's chorea disease, amyotrophic lateral sclerosis, spinocerebellar degenerative disease, Parkinson's disease, and the like. A substance which suppresses the interaction of CGI-94 with Smac/DIABLO may be a substance obtainable by means of screening as described above. Substances which suppresses the interaction of CGI-94 with Smac/DIABLO can be any kind of molecules, including, for example, proteins, peptides, small molecules such as other low molecular-weight organic compounds.

The inventors of the present invention have found a p18TFP protein and its coding DNA as a substance which suppresses the interaction of CGI-94 with Smac/DIABLO, by employing the above-described screening.

In still another aspect, the present invention relates to a method for the treatment of a disease associated with apoptosis of nerve cells, characterized by administering to a subject a substance which suppresses the interaction of CGI-94 with Smac/DIABLO, for example, a p18TFP protein. In addition, the present invention also relates to a pharmaceutical composition for the treatment of a disease associated with apoptosis of nerve cells, comprising a substance which suppresses the interaction of CGI-94 with Smac/DIABLO and pharmaceutically acceptable carriers or excipients. Methods for manufacturing pharmaceutical compositions, dosage forms, and pharmaceutically acceptable carriers or excipients are selected depending upon conditions of subjects, sites to be treated, routes of administration, and the like, and can be readily selected by those skilled in the art. Also, the present invention provides a method for the treatment of apoptosis by gene therapy by which a DNA coding for a p18TFP protein is administered to cells of a subject to express the p18TFP protein, thereby suppressing the interaction of CGI-94 with Smac/DIABLO.

The present invention further relates to a substance which promote the interaction of CGI-94 with Smac/DIABLO. In addition, the present invention also relates to a method for screening substances which promote the interaction of CGI-94 with Smac/DIABLO. Examples of methods for screening substances which promote the interaction of CGI-94 with Smac/DIABLO include methods comprising inserting genes coding for a CGI-94 protein and for a Smac/DIABLO protein into an appropriate vector, transforming a nerve cell using it, and then culturing the transformed cell under the action of a differentiation inducing factor in the presence of a test substance, assaying neurite elongation and apoptosis, and comparing to the result obtained in the absence of the test substance. When in the presence of a differentiation inducing factor at higher concentrations than employed in screening substances which suppress the interaction of CGI-94 with Smac/DIABLO, a test substance is added to a nerve cell having neurite elongation or a cell causing no apoptosis, whereby the growth of neurites is suppressed (in the case of a nerve cell) or apoptosis is observed, it is likely that the test substance is a substance which enhances the interaction of CGI-94 with Smac/DIABLO to promote apoptosis. Vectors may be used for inserting the CGI-94 and Smac/DIABLO genes to transform a nerve cell, methods for transformation, and cells to be used can utilize those as described above. Conditions for culturing cells are determined depending upon respective cells, and are within the knowledge of those skilled in the art or can be readily determined. The nucleotide sequence of the CGI-94 gene and the amino acid sequence of the CGI-94 protein are known (Eur. J. Neurosci. 15, 79 (2001)), and the nucleotide sequence of the Smac/DIABLO gene and the amino acid sequence of the Smac/DIABLO protein are also known (Cell, 102, 33 (2000); Cell, 102, 43 (2000)). It is desirable to attach a detectable tag to a CGI-94 or Smac/DIABLO protein to be expressed, so that the expression of the CGI-94 or Smac/DIABLO protein can be detected with ease. Detectable tags include green fluorescent protein, red fluorescent protein, myc, or the like: fusions with green fluorescent protein, in particular, are simple and sensitive and thus may be recommended.

As described above, a substance which promotes the interaction of CGI-94 with Smac/DIABLO results in promoting of neurite-elongation suppression and of apoptosis, and is useful for establishing apoptosis systems, various studies relating to apoptosis, elucidating the mechanism of diseases associated with apoptosis of nerve cells, for example, nerve-cell death in Alzheimer's disease, and the like. A substances promoting the interaction of CGI-94 with Smac/DIABLO promotes apoptosis, and it is likely that the substance results in rapid enhancement of cell apoptosis and is useful for treating diseases, in particular, diseases resulting from cell hyperproliferation such as cancers and autoimmune disorders. A substance which promotes the interaction of CGI-94 with Smac/DIABLO may be a substance obtainable by means of screening as described above. Substances which promote the interaction of CGI-94 with Smac/DIABLO can be any kinds of molecules, including, for example, proteins, peptides, small molecules such as other low molecular-weight organic compounds.

The present invention also relates to a method for the treatment of a disease resulting from cells, in particular, from cell hyperproliferation, such as a cancer or an autoimmune disorder, characterized by administering to a subject a substance which interacts with CGI-94 to promote its function. In addition, the present invention also relates to a pharmaceutical composition for the treatment of a disease resulting, from cells, in particular, from cell hyperproliferation, such as a cancer or an autoimmune disorder, comprising a substance which interacts with CGI-94 to promote its function and pharmaceutically acceptable carriers or excipients. Methods for manufacturing pharmaceutical compositions, dosage forms, and pharmaceutically acceptable carriers or excipients are selected depending upon conditions of subjects, sites to be treated, routes of administration, and others, and can be readily selected by those skilled in the art.

In still another aspect, the present invention relates to a method for the treatment of a disease resulting from cells, in particular, from cell hyperproliferation, such as a cancer or an autoimmune disorder, characterized by administering to a subject a substance which promotes the interaction of CGI-94 with Smac/DIABLO. In addition, the present invention also relates to a pharmaceutical composition for the treatment of a disease resulting from cells, in particular, from cell hyperproliferation, such as a cancer or an autoimmune disorder, comprising a substance which promotes the interaction of CGI-94 with Smac/DIABLO and pharmaceutically acceptable carriers or excipients. Methods for manufacturing pharmaceutical compositions, dosage forms, and pharmaceutically acceptable carriers or excipients are selected depending upon conditions of subjects, sites to be treated, routes of administration, and others, and can be readily selected by those skilled in the art.

The present invention further relates to a method for examining apoptosis of cells, in particular, nerve cells, and to a method for the diagnosis of a disease associated with such apoptosis, which comprises determining the level of expression of the CGI-94 gene or the CGI-94 protein in cells or tissues. One may detect the CGI-94 gene in sampled cells and tissues obtained from a subject, for example, at the mRNA level, or the CGI-94 protein in sampled cells. Such detection can be achieved by procedures well known to those skilled in the art, such as hybridization employing a probe (preferably, labeled with a radioisotope, fluorophore, enzyme, or the like, for example) complimentary to the CGI-94 gene, or by utilizing the binding to a monoclonal antibody (preferably, labeled with a radioisotope, fluorophore, enzyme, or the like, for example) directed to the CGI-94 protein. In this case, it is possible to determine the intensity of expression of the CGI-94 gene or the amount of the CGI-94 protein by examining the signal intensity of the label.

The present invention also relates to a kit for examining apoptosis in cells, particularly in nerve cells, and to a kit for the diagnosis of diseases associated with such apoptosis, wherein these kits are characterized by examining the level of expression of the CGI-94 gene or CGI-94 protein in cells and tissues. As components of the kit are included, for example, a probe (preferably, labeled with a radioisotope, fluorophore, enzyme, or the like, for example) complimentary to the CGI-94 gene and/or a monoclonal antibody (preferably, labeled with a radioisotope, fluorophore, enzyme, or the like, for example) directed to the CGI-94 protein. The kit usually has its instructions for operation appended thereto.

### EXAMPLES

### Example 1: Actions of CGI-94 and Screening of Proteins Interacting with CGI-94

### A. Methods

### 1. ProQUEST™ Two-Hybrid System by Gateway™ Technology

A yeast strain MaV203 was used. Human CGI-94 was subcloned from pENTR/D-TOPO® into the pDEST™32 vector (Invitrogen) having a GAL4 DNA binding domain. As a vector for expressing an activation domain which has a ProQuest™ two-hybrid human brain cDNA library (Invitrogen), pEXPAD502 was used. Three reporter genes, HIS3, URA3, and lacZ, were used for selection. Each of these genes was integrated stably into a yeast gene at a different site, and the promoter regions of the HIS3, URA3, and lacZ are different except for the GAL4 binding site. It is reported that the ProQuest™ Two-Hybrid System enables three independent transcriptions to take place from respective separate chromosomes, thereby giving reduced false-positive reactions, as compared to standard two-hybrid systems. The HIS3 and URA3 reporter genes are induced depending upon the two-hybrid, and respectively enable cells to grow even on a plate lacking histidine or uracil, so that cells can be discriminated. On the other hand, the LacZ gene can be induced with X-gal (5-brome-4-chloro-3-indolyl-β-D-galactopyranoside), resulting in blue color. In addition, due to the toxicity resulting from the conversion of 5-fluoroorotic acid (5-FOA) to fluorouracil, the induction of URA3 enables cells to grow in a culture medium lacking uracil and inhibits the growth in a culture medium containing 5-FOA. Therefore, this system enables the screening of four phenotypes, that is, proteins displaying the true interaction by means of four phenotypes, His (3AT®), β-gal, Ura⁺ and 5-FOA^{s} and thus the elimination of false-positive reactions. The use of the ARS/CEN vector also can reduce the expression level and the toxicity. Positive clones can be identified by re-transformation. When an interacting protein is contained, a yeast cell binds to DB-human CGI-94 and AD-Y (wherein Y is, for example, Smac). The plasmid DNA from a yeast strain containing DB-human CGI-94 and AD-Y (wherein Y is, for example, Smac) can be introduced into an E. coli by electroporation, and transformants containing AD-Y (wherein Y is, for example, Smac) can be selected with ampicillin, whereas transformants containing the DB-human CGI-94 can be selected with gentamycin. The plasmid DNA from these E. coli, AD-Y (wherein Y is, for example, Smac), can be introduce into MaV203 together with pDBleu or DB-human CGI-94, and the induction of the reporter genes by DB-human CGI-94 will give a true positive reaction.

### 2. Gene Transfer

In order to express transiently in PC12 cells a gene of a protein having GFP (green fluorescent protein) attached at the C-terminal of CGI-94 (CGI-94-CT-GFP), a GFP expression vector or a plasmid lacking it was introduced, using a SuperFector Gene Transfer reagent (B-Bridge, San Jose, USA), into a cell, which was cultured in a D-MEM/F12 (1/1) /N2 medium containing 10% FCS (Gibco) at 37°C in 5% CO₂/95% air. 48 hours after gene transfer, observations were made of survival of cells expressing CGI-94-CT-GFP, employing a fluorescent microscope (Olympus IX70). In addition, 24 hours after gene transfer, NGF (50 ng/ml) was added, and 120 hours later, observations under a fluorescent microscope were made again of neurite elongation and cell survial.

### 3. Cell Culture

PC12 cells were cultured in Dulbecco's Modified Eagle Medium (D-MEM)/F12(1/1) containing N2 supplements and 10% FCS (Gibco) at 37°C in 5% CO₂/95% air.

Those skilled in the art could carry out experiments by other methods than those described above, and various procedures which have not been explained above are usual to those skilled in the art.

### B. Results

1. CGI-94 gave rise to neurite-elongation suppression and apoptosis in PC12 cells whose differentiation had been induced by NGF.

For the purpose of investigating functions of CGI-94 in nerve cells, PC12 cells were subjected to expression of CGI-94-GFP. As a result, in cells expressing it, 120 hours after NGF (50 ng/ml) was added, the suppression of neurite elongation and despite the presence of NGF, apoptosis were able to be seen and two weeks later, all the cells were dead as shown in Fig. 1b. In contrast, cells not expressing the gene (displaying no fluorescence) clearly promoted neurite elongation and survived. Fig. 1a shows the cells without adding NGF added cells 48 hours after the expression: the cells resulted in observing no elongation of neurites, since NGF was not added.

2. Screening for a protein(s) interacting with CGI-94 was performed employing the Two-Hybrid system (whose procedures are as described above) , with the result that such a protein was found to interact strongly with Smac/DIABLO.

### Example 2: Cloning of p18TFP Gene and the Amino Acid Sequence of p18TFP Protein

### A. Methods

### 1. RT-PCR

A p18TFP cDNA was screened and found by a 5'-RACE-RT-PCR method employing a rat brain cDNA library (pAP3neo, TaKaRa, Otsu, Shiga, Japan). RT-PCR methods were applied to analysis of mRNA expression and isolation of p18TFP. Total RNA was prepared according to the TRIzol® Reagent protocol (Gibco BRL, Grand Island, NY, USA). Briefly, after extraction of mRNA with chloroform, the RNA was precipitated by adding an equal volume of isopropyl alcohol, rinsed with 75% ethanol, and dissolved in RNase-free water to measure the absorbance (at 260 nm) on a spectrophotometer. Total RNA of each sample (0.2 µg/ml) was first subj ected to reverse transcription to cDNA (oligo(dT)-primed-SMART™ cDNA-synthesis (Clontech, Tokyo, Japan); Superscript II™ (Gibco BRL, NY, USA)), and then PCR reactions (reaction volume, 25µl) were carried out using as rat cDNA/p18TFP specific primers the following primers: for isolation, firstly 1) sense: 5'-gcg taa tac gac tca cta tag gga att cga cgt-3' (SEQ ID NO:3), antisense: 5'-cgc gac gta cga ttt aaa tta acc ctc act aaa-3' (SEQ ID NO:4), and secondly 2) sense: 5'-gtg cgt tgt cct cgc taa gga cct gaa cga aac gcg atc ttg aaa-3' (SEQ ID NO:5), antisense: 5' -aag ttc tgt gct tta gtt caa aca act gat aac act cta aac aag-3' (SEQ ID NO:6), and 3) for OFR subclonig, sense: 5'-atg gca gtc cca ggt tgc aac aag gac aat gtc c-3' (SEQ ID NO:7), antisense: 5'-tta ctt ttt ggt gac ttc aga acg gtt agg gga aga a-3' (SEQ ID NO:8). The number of reaction cycles employed for amplification of each cDNA was set in the linear range according to the Elongase™ enzyme mix protocol. Denaturing in the amplification step was carried out at 94°C for 0.5 minutes, and annealing was carried out, with the specific primers, at 65°C for 50 seconds, and extension was carried out at 68°C for 1 to 2 minutes (AnnT: 65°C/24 cycles). PCR reactions (AnnT: 60°C/16 cycles) of a constitutively expressing ribosomal protein S12 cDNA were used for measurement of introduced RNA. Controls without reverse transcription employing RNA samples or without RNA were used to ascertain the absence of contamination with DNA. PCR reactions were analyzed by electrophoresis on 1.5% agarose gel.

### 2. Analysis of p18TFP cDNA Sequence and Amino Acid

Analysis of the p18TFP cDNA and amino acid sequences was carried out using PDB, SwissProt, PIF, and PRF, in addition to the NCBI (National Center for Biotechnology Information) Blastp 2.0 program (Nucleic Acids Res., 25, 3389 (1997)) to un-overlapped GenBank CDS translations and the UniGene database (NCBI) (Nucleic Acids Res., 25, 2289 (1997)). Homology search was carried out using Blast and FASTA (Wisconsin Package version 10.0, Genetics Computer Group (GCG), Madison, WI) algorithms, and BestFit (Eisconsin Package 10.0, GCG). Motifs of the amino acid sequence were searched using PROSITE Profile, and BLOCKS, ProDom, PRINTS, Pfam and PSORTII programs (Nucleic Acids Res., 27, 260 (1999); Intellig. Syst. Mol. Biol., 4, 109 (1996); Intellig. Syst. Mol. Biol., 5, 147 (1997)). Phosphorylation sites were searched using NetPhos 2.0 (J. Mol. Biol., 294, 1351 (1999)), and other analyses were. carried out using not only the ExPASy-www server (http://www.expasy.ch), but also http://www/softberry.com/index.html and the amino acid composition search (AACompIdent) http://kr.expasy.org/tools/aacomp/.

### 3. Cell Culture

PC12 cells (for example, available from ATCC (American Type Culture Collection), ATCC No. CRL-1721) were cultured in Dulbecco's Modified Eagle Medium (D-MEM)/F-12 (1:1) containing N2 and supplemented with 10% fetal calf serum (FCS; Gibco BRL, Grand Island, NY, USA), and CHO cellw were cultured in D-MEM supplemented with 10% FCS under the condition of 37°C and 5% CO₂/95% air.

### 4. Gene Transfer into Cells

A p18TFP cDNA was found by gene co-transfer of pCGI-94 and a rat brain cDNA library into rat PC12 cells. Cells were subjected to transient transfection using the SuperFector Gene Transfer reagent (B-Bridge, San Jose, USA) and cultured in a D-MEM/(F12(1:1)/N2) medium containing 10% FCS (Gibco) under the condition of 37°C and 5% CO₂/95% air.

### B. Results

### 1. Isolation of p18TFP

After gene co-transfer of pCGI-94 and a rat brain cDNA library, a gene implicated in survival was found from surviving cells after 6 day culturing, by RT-PCR method, employing the death trap screening method (Semin. Immunol., 9, 17 (1997)), and was named p18TFP. Also, a cell-death suppressing protein encoded by this gene was named p18TFP.

### 2. Characterization of p18TFP

Fig. 2a shows the base sequence of a p18TFP cDNA (2240 nucleotides (nts) with an ORF from nt 140 to nt 601), and Figure 2b shows the amino acid sequence consisting of 153 amino acids (molecular weight, 18 kDa). Since the amino acid sequence of p18TFP contains C2HC-Zn finger and dual nuclear localization signal (NLS) sequence motifs, p18TFP has been shown to have properties as a transcription factor protein family.

According to the present invention, apoptosis suppressing or promoting substances, their screening, and methods for diagnosiss and treatment of diseases associated with apoptosis, and kits therefore are provided. More specifically, according to the present invention substances which interact with CGI-94 causing apoptosis and suppress its function, substances which suppress the interaction of CGI-94 with Smac/DIABLO, methods for screening them, pharmaceutical compositions for treating diseases associated with apoptosis comprising them, methods for treating diseases associated with apoptosis, and methods and kits for diagnosing such diseases are provided. In particular, a p18TFP protein was found as a substance which interacts with CGI-94 causing apoptosis to suppresses its function, or as a substance which suppresses the interaction of CGI-94 with Smac/DIABLO. Also there are provided substances which interact with CGI-94 causing apoptosis to promote its function, substances which promote the interaction of CGI-94 with Smac/DIABLO, and methods for screening them, and these substances will serve for elucidating the mechanism of apoptosis, the mechanism of developing diseases associated with apoptosis such as Alzheimer' s disease, and others.

## Claims

1. A substance which interacts with CGI-94 to suppress apoptosis.

2. A substance which suppresses the interaction of CGI-94 with Smac/DIABLO to suppress apoptosis.

3. A DNA **characterized by**:
(a) a DNA comprising the base sequence of base numbers 140 to 601 of the base sequence of SEQ ID NO:1,
(b) a DNA hybridizable under stringent conditions with a DNA comprising the sequence of (a), or
(c) a DNA having a homology of not less than 60% to the DNA sequence of (a).

4. A protein **characterized by**:
(a) a protein comprising of amino acids 1 to 153 of the amino acid sequence of SEQ ID NO:2 and suppressing apoptosis by interacting with CGI-94 or by suppressing the interaction of CGI-94 with Smac/DIABLO,
(b) a protein having one or more amino acids deleted, substituted, or added in the amino acid sequence of (a) and suppressing apoptosis by interacting with CGI-94 or by suppressing the interaction of CGI-94 with Smac/DIABLO, or
(c) a protein having a homology of not less than 60% to the amino acid sequence of (a).

5. A DNA coding for the protein according to claim 4.

6. A RNA which is complementary to the DNA according to claim 3 or 5.

7. A substance which interacts with CGI-94 to promote apoptosis.

8. A substance which promotes the interaction of CGI-94 with Smac/DIABLO to promote apoptosis.

9. A method for screening substances interacting with CGI-94 to suppress its function, which comprises determining neurite elongation or apoptosis of a cell expressing CGI-94 in the presence of a test substance and a differentiation inducing factor.

10. A method for screening substances interacting with CGI-94 to promote its function, which comprises determining neurite elongation or apoptosis of a cell expressing CGI-94 in the presence of a test substance and a differentiation inducing factor.

11. A substance interacting with CGI-94 to suppress.its function, wherein the substance is found by means of the method of claim 9.

12. A substance interacting with CGI-94 to promote its function, wherein the substance is found by means of the method of claim 10.

13. A method for screening substances suppressing the interaction of CGI-94 with Smac/DIABLO, which comprises determining neurite elongation or apoptosis of a cell expressing CGI-94 and Smac/DIABLO in the presence of a test substance and a differentiation inducing factor.

14. A method for screening substances promoting the interaction of CGI-94 with Smac/DIABLO, which comprises determining neurite elongation or apoptosis of a cell expressing CGI-94 and Smac/DIABLO in the presence of a test substance and a differentiation inducing factor.

15. A substance suppressing the interaction of CGI-94 with Smac/DIABLO, wherein the substance is found by means of the method of claim 13.

16. A substance according to claim 12 or 15, which is a p18TFP protein.

17. A substance promoting the interaction of CGI-94 with Smac/DIABLO, wherein the substance is found by means of the method of claim 14.

18. A pharmaceutical composition for treating a disease associated with apoptosis, comprising a substance interacting with CGI-94 to suppress apoptosis.

19. A pharmaceutical composition for treating a disease associated with apoptosis, comprising a substance suppressing the interaction of CGI-94 with Smac/DIABLO to suppress apoptosis.

20. A pharmaceutical composition according to claim 18 or 19, wherein the substance is a p18TFP protein or a p18TFP DNA.

21. A pharmaceutical composition for treating a disease resulting from cell hyperproliferation, comprising a substance interacting with CGI-94 to promote apoptosis.

22. A pharmaceutical composition for treating a disease resulting from cell hyperproliferation, comprising a substance promoting the interaction of CGI-94 with Smac/DIABLO to promote apoptosis.

23. A method for diagnosis of a disease associated with apoptosis, which comprises determining the level of expression of the CGI-94 gene or the CGI-94 protein in cells or tissues.

24. A kit for diagnosis of a disease associated with apoptosis, which comprises determining the level of expression of the CGI-94 gene or the CGI-94 protein in cells or tissues.


SEQUENCE LISTING

<110> Klaus Heese,

Yasuo Nagai,

Tohru Sawada,

BF Research Institute, Inc.

<120> APOPTOSIS PROMOTING AND SUPPRESSING SUBSTANCES INTERACTING WITH CGI-94,

AND METHODS FOR SCREENING THE SAME

<130> 663697

<150> JP 2002-041176

<151> 2002-02-19

<160> 8

<210> 1

<211> 2240

<212> DNA

<213> rat

<400> 1

ttcgccgant ggtccggact cggntcccta gtacggccgc cntgtgctgg attcgccctt 60

gtgcgttgtc ctcgctaagg acctgaacga aacgcgatct tgaaagctca ggactccggt 120

gaccccactc acctgcatta tggcagtccc aggttgcaac aaggacaatg tccgagcagg 180

ctgcagaaaa tgtggctacc ctggtcatct gacttttgag tgccgcaatt ttcttcgggt 240

agaccccaaa agggacattg tgttggacgt cagcagcaca agcagtgagg acagtgacga 300

agaaagcgag gagctgaaca agcttcaggc tctgcaggag aaaagaataa atgaagaaga 360

agagaagaaa aaggaaaaaa gcagggagaa aattaaattg aaaaaaaaga ggaaaaggtc 420
taactccagc accactgaag aggactcttc aaaacaaaag aaacagaagt atcagaaaaa 480
agaaaagaaa aaggaaaaaa agaacaaatc caaaaaagga aaacatcaca aaaaggaaaa 540
aaagaagaga aaaaaggaaa agcgttcttc ccctaaccgt tctgaagtca ccaaaaagta 600
acagagtctt gggcctaact caggaaattg aagaattcag tttttttaag tattgaacct 660
tccttggaat tgctatataa ttacgctttg cagaaaaatc ccagcctctt ccataaagac 720
gtttttcacc tcctggggcc cttccctccc tgcccgtctg tattaaaccc atctccttac 780
ttgacagctt gtcccaagtc tagatgtgtg cactcacaga cacatcactt tggctgtttg 840
tgtctcggtg tctagctgtc tcggtaagtt aattagcacg aggctttcct gcgatcaaag 900
gagtaatcta catggcaact cctgagtctt gtctgccttc cagtggtaaa gctgcctcgg 960
cggtagtgat tcgctcttga tactgaatag ctttatgtac tctgtaatgt atggaatagt 1020
gaagctcttc actattgttt taaacctcta gactttttaa ataacatcac tgaggataaa 1080
accactttac tgatcttaaa tagtaagttt ttaaatgagt gggatctaca gaagataata 1140
atgaaagtat ttataacagt gtcattgttt tatgaaatgc ttgtttctgg cacttgcctc 1200
ccatgatttt acaacaatta aaacagccct gtttttaaaa caatcatcta gcttactaga 1260
aagtctggca gggagattct gtgctggtcc agactattag ggtagatgct gggttgatct 1320
acaaatgggc cacattggca tatcttaggc gatacctcaa acacagggct ctctgtgtgg 1380
taacagcaaa gtcctcatta caactgattt ctactatgtc cgtgtatgca atacttgcat 1440
atatgtgtat tatggcagtt tttaatgaat gtttgcatat atatttattt tttctgaaag 1500
ggttttagag tgagtttcac actgaacttc tggcttcgtg gataatcaat gttgatattt 1560
tctctttcac ttcagtcaca gatcttagta gcactgtgag aatttttaaa ctaagatttt 1620
catcccgtac tttttaaaat tgaagacttg gaggtagcac catgaaagcc tttgtatagt 1680
aatgaagttt gtatataaat aagaatgttc agtgacatgt tagcggattt aagtgtttct 1740
tacattttta acttcatggt catcagtgta gtttaacatc tagtattccc taatgccagc 1800
atgttaactt actgtgccat aaaagcattg aaaaccaacc tgttatcata ggacagctcc 1860
agagtttctg agtgccttgt tatgctgcac ttaatacatc ttccgattgg taagtacact 1920
tgtgctgggc aaatttatgg ccataaaatc agatacatac acgtggcctt gcaggccgag 1980
agctttacca attttcccct tacccagcac ccaaactgac atttcctgat tgcctcaggg 2040

acgcctggga agtcccctaa caccctcgtc tctctctgct caggttgcag agaagctgca 2100
gcacagcctt ccacacaccg ctccctcaca ctgacgtacg tgcagcttcc gctcttccac 2160
aaactggcta ctctcccagc ccatagaaac gtaatcttgt ttagagtgtt atcagttgtt 2220
tgaactaaag cacagaactt 2240

<210> 2
<211> 153
<212> PRT
<213> rat

<400> 2
Met Ala Val Pro Gly Cys Asn Lys Asp Asn Val Arg Ala Gly Cys Arg
1               5                   10                  15
Lys Cys Gly Tyr Pro Gly His Leu Thr Phe Glu Cys Arg Asn Phe Leu
            20                  25                  30
Arg Val Asp Pro Lys Arg Asp Ile Val Leu Asp Val Ser Ser Thr Ser
        35                  40                  45
Ser Glu Asp Ser Asp Glu Glu Ser Glu Glu Leu Asn Lys Leu Gln Ala
    50                  55                  60
Leu Gln Glu Lys Arg Ile Asn Glu Glu Glu Glu Lys Lys Lys Glu Lys
65                  70                  75                  80
Ser Arg Glu Lys Ile Lys Leu Lys Lys Lys Arg Lys Arg Ser Asn Ser
                85                  90                  95
Ser Thr Thr Glu Glu Asp Ser Ser Lys Gln Lys Lys Gln Lys Tyr Gln
            100                 105                 110
Lys Lys Glu Lys Lys Lys Glu Lys Lys Asn Lys Ser Lys Lys Gly Lys
        115                 120                 125

His His Lys Lys Glu Lys Lys Lys Arg Lys Lys Glu Lys Arg Ser Ser
    130                 135                 140
Pro Asn Arg Ser Glu Val Thr Lys Lys
145                 150

<210> 3
<211> 33
<212> DNA
<213>

<400> 3
gcgtaatacg actcactata gggaattcga cgt 33

<210> 4
<211>
<212> DNA
<213>

<400> 4
cgcgacgtac gatttaaatt aaccctcact aaa 33

<210> 5
<211>
<212> DNA
<213>

<400> 5

gtgcgttgtc ctcgctaagg acctgaacga aacgcgatct tgaaa 45

<210> 6

<211>

<212> DNA

<213>

<400> 6

aagttctgtg ctttagttca aacaactgat aacactctaa acaag 45

<210> 7

<211>

<212> DNA

<213>

<400> 7

atggcagtcc caggttgcaa caaggacaat gtcc 34

<210> 8

<211>

<212> DNA

<213>

<400> 8

ttactttttg gtgacttcag aacggttagg ggaagaa 37